# EUROPEAN PATENT SPECIFICATION

(11) **EP 2 961 753 B1**
(45) Date of publication and mention of the grant of the patent: **19.04.2017**
(21) Application number: 15764747.0
(22) Date of filing: 13.03.2015
(51) Int. Cl.: C07D 491/10, A61K 31/445

(54) **STANNOUS FLUORESCENT PROBE**
ZINNFLUORESZENZSONDE
SONDE FLUORESCENTE STANNEUSE

(30) Priority: 20.03.2014 WO PCT/CN2014/073769
(43) Date of publication of application: 06.01.2016
(73) Proprietor: The Procter & Gamble Company, Cincinnati, OH 45202 (US)
(72) Inventor: SHI, Yunming, Beijing 101312 (CN); STRAND, Ross, Singapore 138547 (SG); YI, Tao, Shanghai 200433 (CN); LAN, Haichuang, Shanghai 200433 (CN); WEN, Ying, Shanghai 200433 (CN)
(74) Representative: Joos, Uta Susanne
(86) International application number: PCT/CN2015/074142
(87) International publication number: WO 2015/139577

(56) References cited:
- CN-A- 102 516 254
- LIN YUAN ET AL: "A fast-responsive fluorescent probe for detection of gold ions in water and synthetic products", CHEMICAL COMMUNICATIONS, vol. 47, no. 16, 1 January 2011 (2011-01-01), page 4703, XP055169691, ISSN: 1359-7345, DOI: 10.1039/c0cc05585a
- LAN, HAICHUANG ET AL.: 'Fluorescence Turn-on Detection of Sn2+ in Live Eukaryotic and Prokaryotic Cells' ANALYST vol. 139, no. 20, 21 July 2014, ISSN 0003-2654 pages 5223 - 5229, XP055169684
- ADAMCZYK, MACIEJ ET AL.: 'Efficient Fluorescein Spirolactam and Bis-Spirolactam Synthesis' SYNTHETIC COMMUNICATIONS vol. 31, no. 17, 31 December 2001, ISSN 0039-7911 pages 2681 - 2690, XP009120751

## Description

### FIELD OF THE INVENTION

Fluorescent probes selectively chelating Sn²⁺.

### BACKGROUND OF THE INVENTION

Stannous (Sn²⁺) is added to toothpaste to prevent dental plaque and oral disease. Sn²⁺ is found to effectively inhibit certain bacteria that can lead to tooth decay in human interproximal dental plaque. More recently, there has been increasing interest in the biological roles of Sn²⁺ because tin is an essential trace mineral for humans and is found in the greatest amount in the adrenal gland, liver, brain, spleen and thyroid gland. There is some evidence that tin is involved in growth factors and cancer prevention. Deficiency of tin may result in poor growth and hearing loss, but excess tin accumulation can negatively affect respiratory and digestive systems. However, studies of the physiological role and bacteriostatic mechanism of tin ion are restricted by the lack of versatile Sn²⁺ detection methods applicable to living cells - either eukaryotic or prokaryotic.

Lin Yuan et al. disclosed a fast.responsive fluorescent probe for detection of gold ions in water and synthetic products (ChemComm., 2011, 47, 4703-4705). However, there is still a need for chemical probe that is highly selective for Sn²⁺ in the presence of various metal ions and will exhibit high fluorescence upon Sn²⁺ chelation. There is a further need for such probe for use in living cells. There is yet a further need for a probe that minimizes background noise while providing high fluoresce intensity in living cells consistent where Sn²⁺ is found in the cell.

It is an advantage to have a probe that works well in pH conditions in an organelle that plays a role in Sn²⁺ accumulation in the cell.

It is a further advantage to have a probe that is relatively easy and simple to synthesize.

It is an advantage to have a probe that is less toxic, or at least minimizes toxicity, to biological cells so that experiments with living cells may be conducted.

### SUMMARY OF THE INVENTION

The present invention address this need by the surprising discovery of a Sn²⁺ fluorescent probe containing rhodamine B derivative moiety as fluorophore, linked via amide moiety to a carbazate group. The use of this class of probe compounds is demonstrated as an imaging probe for monitoring Sn²⁺ in living cells to study the physiological function of Sn²⁺ in biological systems. This class of compounds is particularly useful given the additional surprising discovery that lysosomes appear to be an organelle where Sn concentrations are found. And given the rather acidic microenvironment of this organelle, the probes of the present invention exhibit high fluorescent intensity and yet minimizes background noise, compared to other probes that are otherwise subject to low pH induced fluoresce. In other words, a comparative probe, given the acidity of the lysosome, leads to undesirably induce fluorescence emission (thereby generating background noise).

A first aspect of the invention provides for a compound having the following Formula (I): 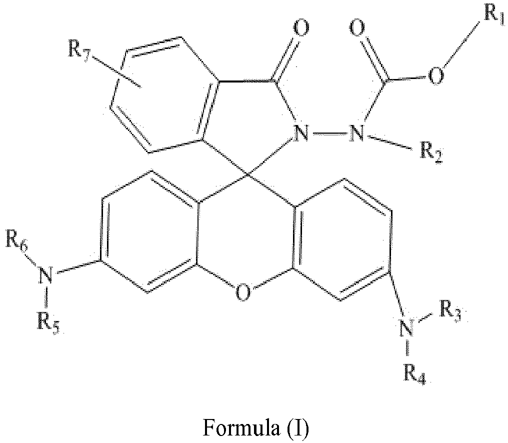 wherein R₁ is unsubstituted, branched or unbranched, C₁-C₁₂ alkyl, alkenyl, or alkynyl; and wherein R₂, R₃, R₄, R₅, R₆, and R₇ are each independently a hydrogen or a hydrocarbyl, heteroalkyl, heteroalkenyl, heteroalkynyl, heterocycloalkyl, heterocycloalkenyl, heteroaryl, and wherein the aforementioned may be substituted or unsubstituted; or an optical isomer, diastereomer or enantiomer for Formula (I), or a salt thereof.

In one embodiment, R₁ is unsubstituted, branched or unbranched, C₁-C₁₀ alkyl, preferably C₁-C₈ alkyl. In another embodiment, R₁ is selected from the group consisting of methyl, ethyl, propyl, butyl, isobutyl, pentanyl, and hexanyl, preferably isobutyl.
In one embodiment, R₂, R₃, R₄, R₅, R₆, and R₇ are each independently selected from the group consisting of H, alkyl, alkenyl, alkynyl, cycloalkyl, cycloalkenyl, aryl, heteroalkyl, heteroalkenyl, heteroalkynyl, heterocycloalkyl, heterocycloalkenyl, heteroaryl, and wherein the aforementioned may be substituted or unsubstituted. In another embodiment, R₂, R₃, R₄, R₅, R₆, and R₇ are each independently selected from H, C₁-C₁₀ alkyl, alkenyl, or alkynyl, and wherein the aforementioned may be substituted or unsubstituted, preferably unsubstituted. In yet still another embodiment, R₂ and R₇ are hydrogen, and/or R₃, R₄, R₅, and R₆ are each independently selected from hydrogen, or C₁ to C₅ alkyl, branched or unbranched, preferably unsubstituted C₁ to C₅ alkyl. In yet still another embodiment, R₃, R₄, R₅, and R₆, are each independently selected from unsubstituted C₁ to C₃ alkyl.

Another aspect of the invention provides a compound of Formula (II): 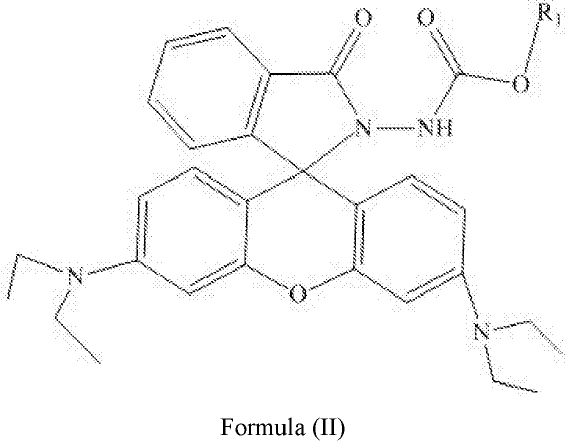 wherein R₁ is unsubstituted, branched or unbranched, C₁-C₁₂ alkyl or alkenyl; or an optical isomer, diastereomer or enantiomer for Formula (I), or a salt thereof. In one embodiment, R₁ is an unsubstituted C₁-C₁₀ alkyl, preferably R₁ is an unsubstituted, branched or unbranched, C₁-C₈ alkyl. In yet still another embodiment, R₁ is selected from the group consisting of methyl, ethyl, propyl, butyl, isobutyl, pentanyl, and hexanyl, preferably isobutyl.

In another aspect of the invention, a compound according to Formula (I) or (II) is provided, wherein the compound is selected from the group consisting of:
(a) Tert-butoxy-carboxamide, N-[3',6'-bis(diethylamino)-3-oxospiro [1H-isoindole-1,9'-[9H]xanthen]-H)-yl]-;
(b) Tert-butoxy-carboxamide,N-[3',6'-bis(dimethylamino)-3-oxospiro[1H-isoindole-1,9'-[9H]xanthen]-2(3H)-yl]-;
(c) Methoxy-carboxamide,N-[3',6'-bis(diethylamino)-3-oxospiro[1H-isoindole-1,9'-[9H]xanthen]-2(3H)-yl]-;
(d) Ethoxy-carboxamide,N-[3',6'-bis(diethylamino)-3-oxospiro[1H-isoindole-1,9'-[9H]xanthen]-2(3H)-yl]-; and
(e) Methoxy-carboxamide,N-[3',6'-bis(dimethylamino)-3-oxospiro[1H-isoindole-1,9'-[9H]xanthen]-2(3H)-yl]-; and
(f) Ethoxy-carboxamide,N-[3',6'-bis(dimethylamino)-3-oxospiro[1H-isoindole-1,9'-[9H]xanthen]-2(3H)-yl]-.

In yet another embodiment, the compound is selected from: N-(3',6'-bis(diethylamino)-3-oxospiro[isoindoline-1,9'-xanthen]-2-yl)propionamide; N-(3',6'-bis(diethylamino)-3-oxospiro[isoindoline-1,9'-xanthen]-2-yl)butyramide; and N-(3',6'-bis(diethylamino)-3-oxospiro[isoindoline-1,9'-xanthen]-2-yl)pentanamide.

Yet still another aspect of the invention provides for a method of detecting fluorescence in a biological cell comprising the steps: (a) incubating the biological cell with a compound described above (e.g., a compound of Formula (I) or Formula (II), or preferred or alternative compound embodiments within said Formulas (I) or (II)); (b) shining excitation light to the incubated cell , preferably wherein the shined light has wavelength of at least from 520 to 580 nm, alternatively at 560 nm; and (c) detecting light emission from the compound from 560 to 660 nm. In one embodiment, the method further comprises subjecting the biological cell to Sn²⁺, alternatively wherein the biological cell is selected from an oral epithelial cell or *Streptococcus* genus of bacterium, alternatively wherein the biological cell is any eukaryotic cell. In another embodiment, the light emission detection is at a lysosome organelle of eukaryotic cell. In yet still another embodiment, the method is conducted with at least one specific compound previously described above or herein.

### BRIEF DESCRIPTION OF THE DRAWINGS

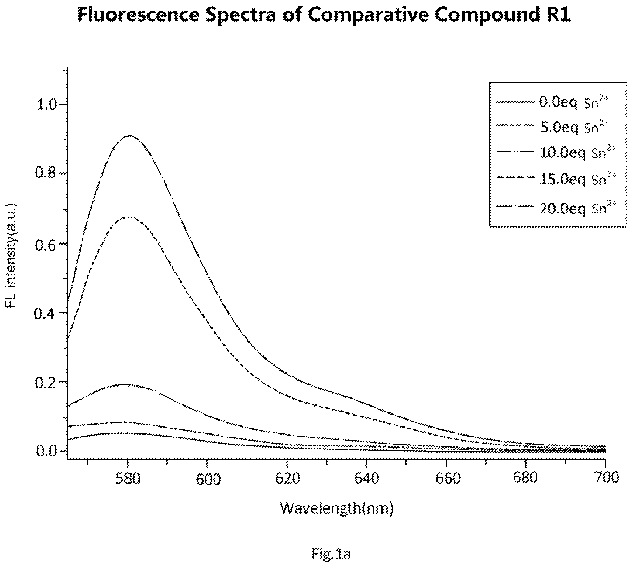

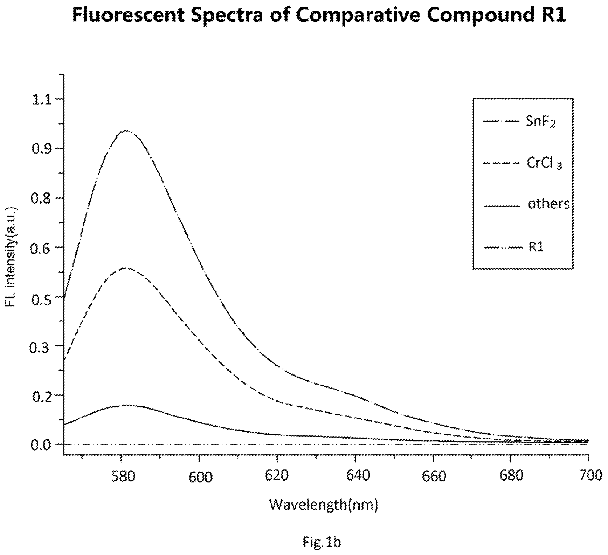

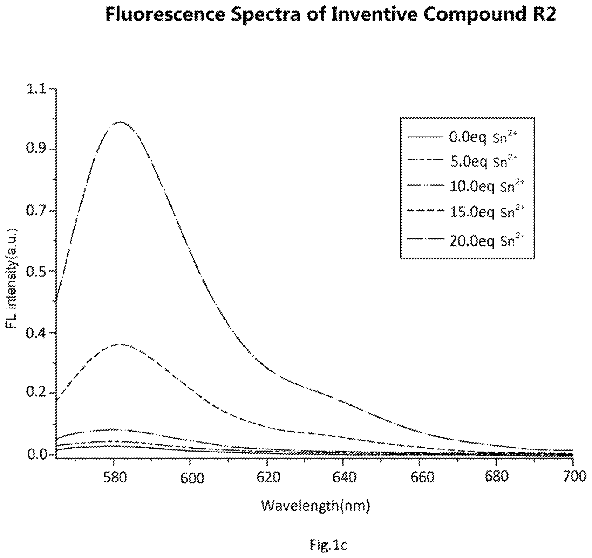

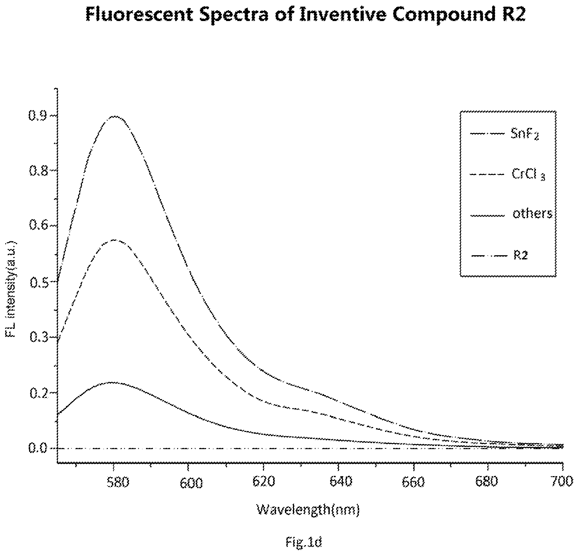

Figures 1 - 5 are provided. Figure 1 (a) and (c) is fluorescence spectra of comparative Compound R1 and inventive Compound R2 upon addition of Sn²⁺; (b) and (d) is fluorescent spectra of Compounds R1 and R2 upon various metal ions with an excitation of 560 nm, respectively. Compound R1 is Spiro[1*H*-isoindole-1,9'-[9*H*]xanthen]-3(2*H*)-one, 2-[2-[bis(2-hydroxyethyl)amino]amino]ethyl]-3',6'-bis(diethylamino). Compound R2 is Tert-butoxy-carboxamide,N-[3',6'-bis(diethylamino)-3-oxospiro[1H-isoindole-1,9'-[9H]xanthen]-H)-yl]-.

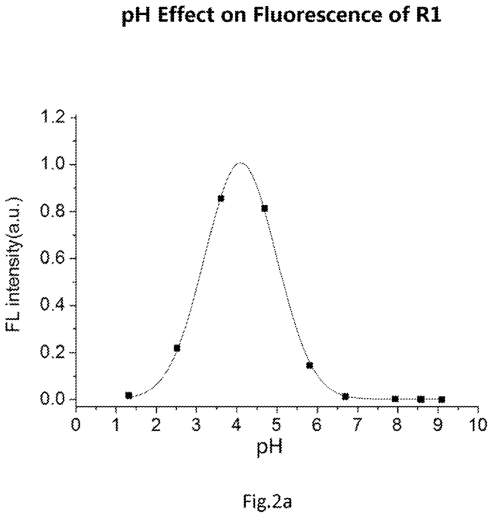

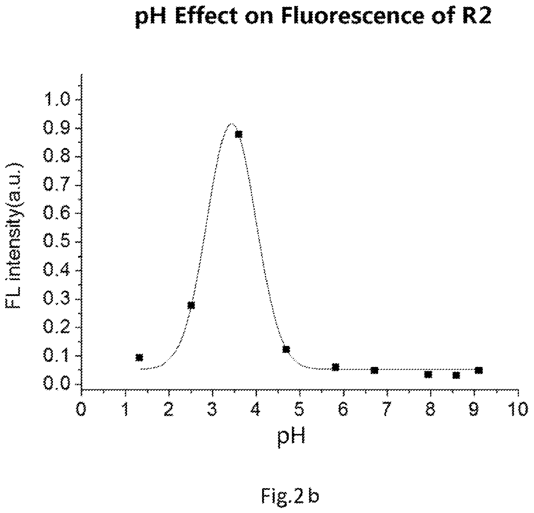

Figures 2a and 2b show dependence of fluorescence at 580 nm of comparative Compound R1 and inventive Compound R2 (10 µM) at different pH range, respectively. Excitation is at 560 nm.

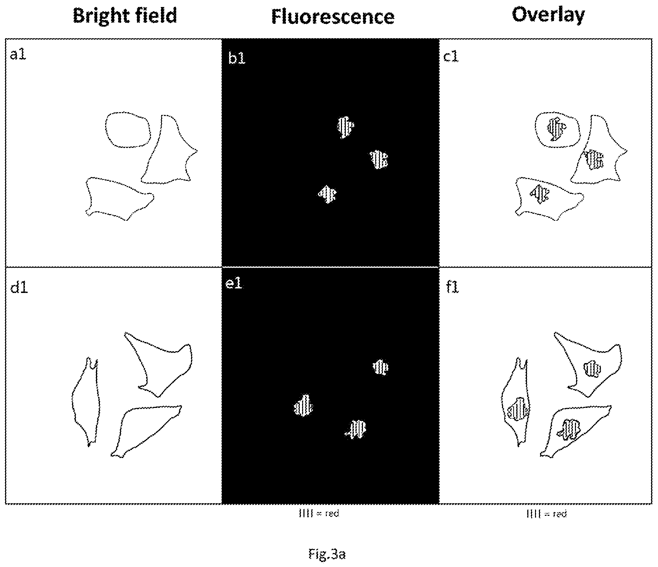

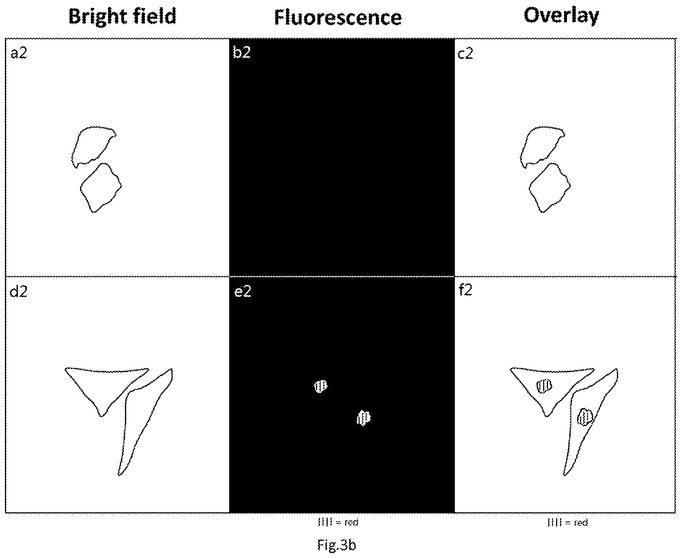

Figures 3a and 3b are simplified illustrations derived based upon CLSM (Confocal Laser Scanning Microscope) images of KB cells (a subline of the ubiquitous Keratin-forming tumor cell line HeLa). (a1-c1) and (a2-c2) cells are separately incubated with 10µM comparative Compound R1 and inventive Compound R2 for 30 min, (d1-f1) and (d2-f2) follows incubation with 50µM SnF₂ for 30 min. Emission is collected in red channel at 560-660 nm (b1, e1, b2, e2); a1, d1, a2, d2 are bright field images and c1, f1, c2, f2 are overlay images, respectively (λex = 543 nm). Any observed fluorescence is red.

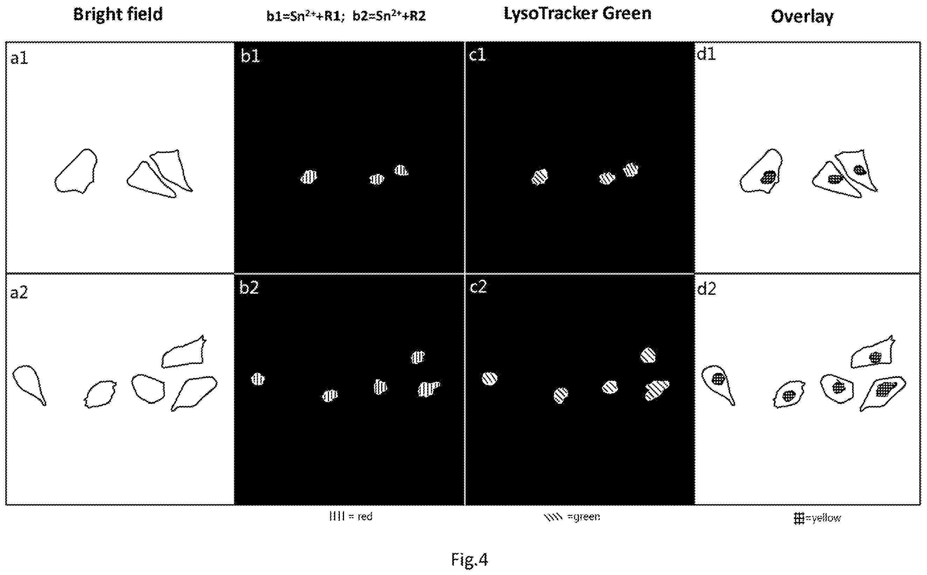

Figure 4 are simplified illustrations derived based upon CLSM images of KB cells of a comparative colocalization experiment. (a1 - d1) cells are successively incubated with 50µM SnF₂, 10 µM of comparative Compound R1 and 1µM LysoTracker® Green DND each for 30 min; (a2-d2) successively incubated with 50µM SnF₂, 10µM of inventive Compound R2 and 1µM LysoTracker® Green DND, each for 30 min. Emission is collected in red channel (b1, b2) at 560-660 nm (λex = 543 nm) or in green channel at 500-540 nm (λex = 488 nm); a1, a2, are bright field images and d1, d2 are overlay images, respectively. Observed fluorescence is: red in b1, b2; green in c1, c2; and yellow in d1, d2.

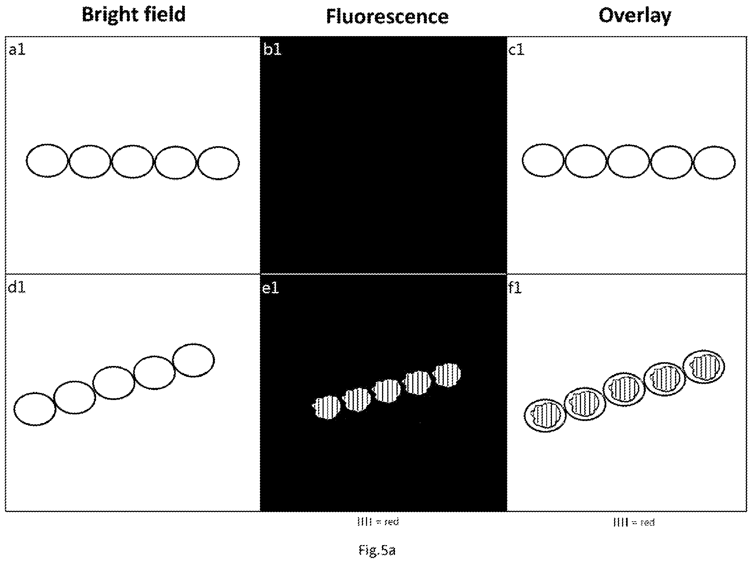

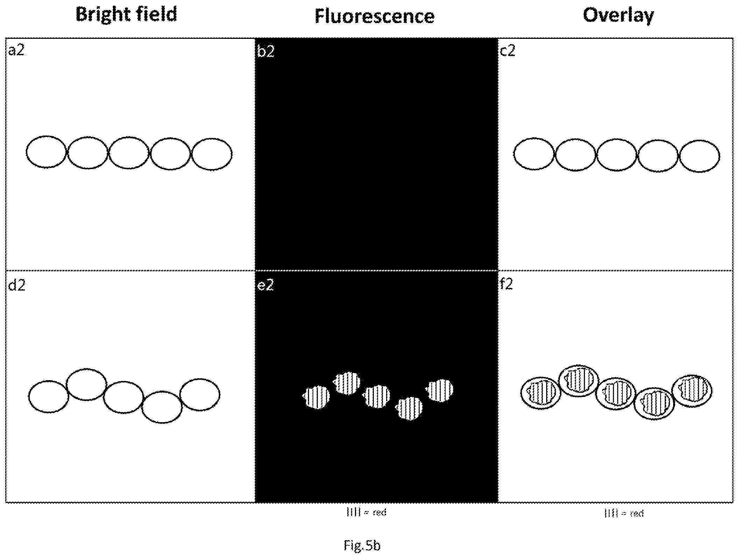

Figures 5a and 5b show simplified illustrations derived based upon CLSM images of *Streptococcus mutans* (ATCC® 700610™). The illustrations are not drawn to scale but rather enlarged for illustrative purposes. (a1-c1) and (a2-c2) Cells are separately incubated with 10µM comparative Compound R1 and inventive Compound R2 for 30 min, (d1-f1) and (d2-f2) followed incubated with 50µM SnF₂ for 30 min. Emission is collected in red channel at 560-660 nm (b1, e1, b2, e2); a1, d1, a2, d2 are bright field images and c1, f1, c2, f2 are overlay images, respectively (λex = 543 nm).

### DETAILED DESCRIPTION OF THE INVENTION

### Definitions:

For purposes of the present invention the term "hydrocarbyl" is defined herein as any organic unit or moiety which is comprised of carbon atoms and hydrogen atoms. Included within the term "hydrocarbyl" are heterocycles. Non-limiting examples of various unsubstituted non-heterocyclic hydrocarbyl units include pentyl, 3-ethyloctanyl, 1,3-dimethylphenyl, cyclohexyl, cis-3-hexyl, 7, 7-dimethylbicyclo[2.2.1]-heptan-1-yl, and napth-2-yl. Included with the definition of "hydrocarbyl" are the aromatic (aryl) and non-aromatic carbocyclic rings. The term "heterocycle" includes both aromatic (heteroaryl) and non-aromatic heterocyclic rings.

The term "substituted" is used throughout the specification. The term "substituted" is defined herein as "encompassing moieties or units which can replace a hydrogen atom, two hydrogen atoms, or three hydrogen atoms of a hydrocarbyl moiety. Also substituted can include replacement of hydrogen atoms on two adjacent carbons to form a new moiety or unit." For example, a substituted unit that requires a single hydrogen atom replacement includes halogen, hydroxyl, and the like. A two hydrogen atom replacement includes carbonyl, oximino, and the like. A two hydrogen atom replacement from adjacent carbon atoms includes epoxy, and the like. Three hydrogen replacement includes cyano, and the like. An epoxide unit is an example of a substituted unit which requires replacement of a hydrogen atom on adjacent carbons. The term "substituted" is used through the present specification to indicate that a hydrocarbyl moiety, *inter alia*, aromatic ring, alkyl chain, can have one or more of the hydrogen atoms replaced by a substituent. When a moiety is described a "substituted" any number of the hydrogen atoms may be replaced. For example, 4-hydroxyphenyl is a "substituted aromatic carbocyclic ring," (N, N-dimethyl-5-amino)octanyl is a "substituted C₈ alkyl unit, 3-guanidinopropyl is a "substituted C₃ alkyl unit," and 2-carboxypyridinyl is a "substituted heteroaryl unit".

In one embodiment, wherein R₂, R₃, R₄, R₅, R₆, and R₇ are each independently selected from the group consisting of H, alkyl, alkenyl, alkynyl, cycloalkyl, cycloalkenyl, aryl, heteroalkyl, heteroalkenyl, heteroalkynyl, heterocycloalkyl, heterocycloalkenyl, heteroaryl, substituted, and wherein the aforementioned may be substituted or unsubstituted. These terms are well known in the art. For a detailed definition, see U.S. Pat. No. 6,919,346 B2 at column 2, line 61 to column 9, line 53, incorporated herein by reference.

### Synthesis path of Tert-butoxy-carboxamide,N-[3',6'-bis(diethylamino)-3-oxospiro[1H-isoindole-1,9'-[9H]xanthen]-H)-yl]-(herein after "Compound R2" or simply "R2")

Compound R2, an exemplary compound of the present invention, is rhodamine B derivative moiety linked via amide moiety to tert-butyl carbazate group. The synthesis path of this compound is provided.

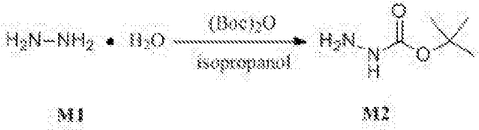

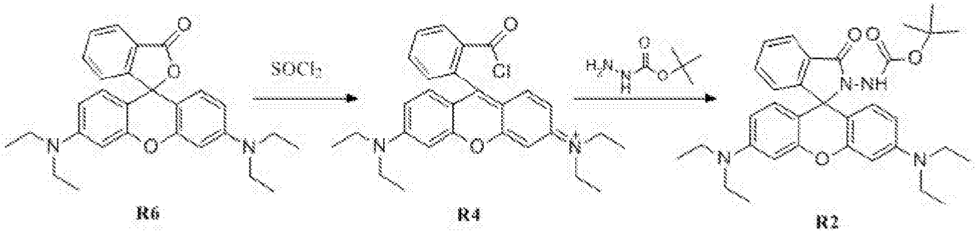

The synthesis of Compound R2 is described. Unless otherwise noted, materials were obtained from commercial suppliers and were used without further purification. Rhodamine (95%) is obtained from Sinopharm Chemical Reagent Co., Ltd. (Shanghai). Other chemicals are provided from Shanghai No. 1 chemical reagent. Flash chromatography is carried out on silica gel (200-300 mesh). The ¹H NMR (500 MHz) and ¹³C NMR (125 MHz) spectra is recorded on a Bruker DRX-500 spectrometer. Proton chemical shifts are reported in parts per million downfield from tetramethylsilane (TMS). HRMS is recorded on LTQ-Orbitrap mass spectrometer (ThermoFIsher, San Jose, CA). Melting points are determined on a hot-plate melting point apparatus XT4-100A and uncorrected. UV-Vis spectra are recorded on a Shimadzu UV-2250 spectrophotometer. Fluorescence spectra are recorded on an Edinburgh FLS-920 spectrophotometer. All pH measurements are made with a model Mettler-Toledo meter.

The synthesis of chemical intermediate M2 (of the schematic above) is described. Hydrazine monohydrate (5.2 g, 100.0 mmol) is stirred in 20 mL of isopropanol at 0° C for 15 min, and treated dropwise with a solution of Boc₂O (Di-tert-butyl dicarbonate)(10.0 g, 45.8 mmol) in 10 mL of isopropanol. The reaction turns cloudy upon addition and stirring is continued at room temperature for 20 min. The solvent is removed by rotary evaporation and the residue is dissolved in DCM (Dichloromethane) and dried over MgSO₄. The DCM is removed by rotary evaporation and the remaining liquid is distilled under reduced pressure to obtain *t*-butyl carbazate (M2) as a white solid, ¹H NMR (400 MHz, CDCl₃) *δ* 6.42 (s, 1H), 3.60 (s, 2H), 1.37 (s, 9H).

Synthesis of chemical intermediate R4 of the above scheme is described. A solution of rhodamine B (442 mg, 1 mmol) in Cl₂SO (10 mL) is kept at room temperature overnight. The reaction mixture is evaporated under vacuum and co-evaporated with anhydrous CH₂Cl₂ (3 × 15 mL) to give rhodamine B acid chloride (R4). The crude acid chloride is dissolved in anhydrous CH₂Cl₂ (10 mL) and added dropwise to a solution of Boc-NH-NH₂ (132 mg, 1 mmol) and Et₃N (200 mL, 2 mmol) in anhydrous CH₂Cl₂ (15 mL). The reaction mixture is kept at room temperature for 10 min. Evaporation of the solvent yielded a crude that is purified by column chromatography using petroleum ether/ethyl acetate (3/1, v/v) to give R2 as a white solid (0.26 g, 50% yield). ¹H NMR (500 MHz, CDCl₃) *δ* 7.96 (d, *J* = 7.6 Hz, 1H), 7.57 - 7.44 (m, 2H), 7.16 (d, *J* = 7.5 Hz, 1H), 6.52 (s, 2H), 6.38 (d, *J* = 1.8 Hz, 2H), 6.28 (d, *J* = 8.4 Hz, 2H), 3.34 (q, *J* = 7.0 Hz, 8H), 1.61 (s, 4H), 1.26 (s, 9H), 1.16 (d, *J* = 14.1 Hz, 12H). ¹³C NMR (125 MHz, CDCl₃) *δ* 168.92, 153.56, 152.87, 148.92, 132.58, 131.45, 129.05, 128.32, 123.80, 123.09, 108.24, 105.41, 97.68, 59.49, 57.65, 54.48, 44.13, 39.49, 12.31. HRMS calc. for C₃₃H₄₁N₄O₄⁺ (M+H⁺): 557.3122, found: 557.3111.

### Comparative Example - Compound R1 or "R1"

Comparative compound R1, outside the scope of the present invention, is compared to inventive Compound R2. Compound R1 is: Spiro[1*H*-isoindole-1,9'-[9*H*]xanthen]-3(2*H*)-one, 2-[2-[bis(2-hydroxyethyl)amino]amino]ethyl]-3',6'-bis(diethylamino), and has the CAS Registry Number of 1217892-36-8 (C₃₄, H₄₄, N₄, O₄). The structure of Compound R1 and its synthesis is provided herein:

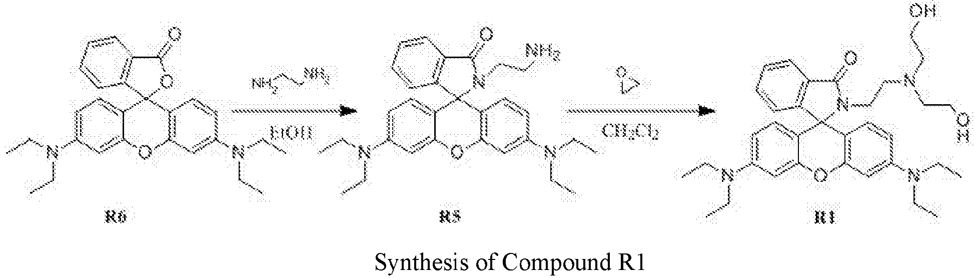

The synthesis of comparative Compound R1 is described. Synthesis of intermediate R5 is described: Rhodamine B hydrochloride (5.0 g, 10.4 mmol) and ethylenediamine (12.5 g, 208.8 mmol) is dissolved in EtOH (50 mL) and refluxed for 12 h. Most of solvent is removed by evaporation, and the residue is dispersed in water with magnetic stirring. Then the pink precipitate appeared and is recovered by filtration, washed thoroughly with water. At last pink precipitate is washed with petroleum ether, and then dried in vacuum, yielding Compound R5 as a pink powder (3.6 g, 72% yield): ¹H NMR (400 MHz, CDCl₃) δ 7.91 (d, *J* = 2.3 Hz, 1H), 7.45 (d, *J* = 2.5 Hz, 2H), 7.14 - 7.05 (m, 1H), 6.44 (s, 1H), 6.42 (s, 1H), 6.37 (d, *J* = 2.3 Hz, 2H), 6.28 (d, *J* = 2.3 Hz, 1H), 6.26 (d, *J* = 2.4 Hz, 1H), 3.33 3 (q, *J* = 7.0 Hz, 9H), 3.19 (t, *J* = 6.6 Hz, 2H), 2.40 (t, *J* = 6.6 Hz, 2H), 1.62 (s, 5H), 1.16 (t, *J* = 7.0 Hz, 13H). *See* Shiraishi, Y.; Miyamoto, R.; Zhang, X.; Hirai, T. Org. Lett. 2007, 9, 3921-3924.

Synthesis of R1 from R5 is described. Oxirane (0.44 g, 10.0 mmol) is added to a cooled (-5° C) solution of R5 (0.48 g, 0.1 mmol) in dichloromethane (10 mL). The solution is stirred for 4 h at -5°C and then overnight at room temperature before being concentrated under reduced pressure. The resulted mixture is purified by column chromatography using dichloromethane/ methanol (10/1, v/v) to give Compound R1 as a white solid (0.2 g, 40% yield): ¹H NMR (500 MHz, CDCl₃) *δ* 7.91 (d, *J* = 2.9 Hz, 1H), 7.46 (dd, *J* = 5.5, 3.1 Hz, 2H), 7.13 - 7.06 (m, 1H), 6.46 (s, 1H), 6.44 (s, 1H), 6.39 (d, *J* = 2.4 Hz, 2H), 6.30 (d, *J* = 2.5 Hz, 1H), 6.28 (d, *J* = 2.5 Hz, 1H), 3.54 - 3.45 (m, 4H), 3.34 (q, *J* = 7.0 Hz, 9H), 3.22 (t, *J* = 5.7 Hz, 2H), 2.55 (t, *J* = 7.5 Hz, 4H), 2.23 (t, *J* = 5.0 Hz, 2H), 1.89 (s, 2H), 1.17 (t, J = 7.0 Hz, 13H). ¹³C NMR (125 MHz, CDCl₃) *δ* 153.96, 150.99, 148.86, 133.12, 129.67, 129.32, 128.31, 124.24, 123.41, 107.85, 104.52, 97.76, 44.10, 27.81, 12.59. HRMS calc. for C34H45N4O4 (M+H⁺): 573.3435, found: 573.3463.

### Derivations of Compound R2

Many further derivations from this basic molecule of Compound R2 can be made by those skilled in the art consistent with Formulas (I) and (II) by using starting materials and intermediates that are known or commercially available or by further modifying these molecules by known methods. Non-limiting examples of these compounds within the scope of Formula (I) and/or Formula (II) including the following:
(1) tert-butyl (3',6'-diamino-3-oxospiro[isoindoline-1,9'-xanthen]-2-yl)carbamate (Chemical Formula: C25H24N4O4), (Molecular Weight: 444.48);
(2) tert-butyl (3',6'-bis(dimethylamino)-3-oxospiro[isoindoline-1,9'-xanthen]-2-yl)carbamate (Chemical Formula: C29H32N4O4), (Molecular Weight: 500.59);
(3) tert-butyl (3',6'-bis(diethylamino)-3-oxospiro[isoindoline-1,9'-xanthen]-2-yl)carbamate (Chemical Formula: C33H40N4O4), (Molecular Weight: 556.70);
(4) tert-butyl (3',6'-bis(ethylamino)-2',7'-dimethyl-3-oxospiro [isoindoline-1,9'-xanthen]-2-yl)carbamate (Chemical Formula: C31H36N4O4), (Molecular Weight: 528.64);
(5) tert-butyl (3',6'-diamino-2',7'-dimethyl-3-oxospiro[isoindoline-1,9'-xanthen]-2-yl)carbamate (Chemical Formula: C27H28N4O4), (Molecular Weight: 472.54);
(6) tert-butyl (3-oxo-3',6'-di(pyrrolidin-1-yl)spiro[isoindoline-1,9'-xanthen]-2-yl)carbamate (Chemical Formula: C33H36N4O4), (Molecular Weight: 552.66);
(7) tert-butyl (3-oxo-3',6'-bis(phenylamino)spiro[isoindoline-1,9'-xanthen]-2-yl)carbamate (Chemical Formula: C37H32N4O4), (Molecular Weight: 596.67);
(8) tert-butyl (3-oxo-3',6'-di(piperidin-1-yl)spiro[isoindoline-1,9'-xanthen]-2-yl)carbamate (Chemical Formula: C35H40N4O4), (Molecular Weight: 580.72);
(9) tert-butyl (3',6'-dimorpholino-3-oxospiro[isoindoline-1,9'-xanthen]-2-yl)carbamate (Chemical Formula: C33H36N4O6), (Molecular Weight: 584.66);
(10) tert-butyl(2',7'-dibutyl-3',6'-bis(diethylamino) -3-oxospiro[isoindoline-1,9'-xanthen]-2-yl)carbamate (Chemical Formula: C41H56N4O4), (Molecular Weight: 668.91);
(11) tert-butyl (2',7'-dimethyl-3-oxo-3',6'-di(piperidin-1-yl)spiro [isoindoline-1,9'-xanthen]-2-yl)carbamate (Chemical Formula: C37H44N4O4), (Molecular Weight: 608.77);
(12) tert-butyl (3-oxo-1',2',3',4',10',11',12',13'-octahydrospiro[isoindoline-1,7'-pyrano [2,3-f:6,5-f]diquinolin]-2-yl)carbamate (Chemical Formula: C31H32N4O4), (Molecular Weight: 524.61);
(13) tert-butyl (3-oxo-1',2',3',4',8',9',10',11'-octahydrospiro[isoindoline-1,6'-pyrano [3,2-g:5,6-g']diquinolin]-2-yl)carbamate (Chemical Formula: C31H32N4O4), (Molecular Weight: 524.61);
(14) N-(3',6'-bis(diethylamino)-3-oxospiro[isoindoline-1,9'-xanthen]-2-yl)propionamide (Chemical Formula: C31H36N4O3), (Molecular Weight: 512.64);
(15) N-(3',6'-bis(diethylamino)-3-oxospiro[isoindoline-1,9'-xanthen]-2-yl)butyramide (Chemical Formula: C32H38N4O3), (Molecular Weight: 526.67); and
(16) N-(3',6'-bis(diethylamino)-3-oxospiro[isoindoline-1,9'-xanthen]-2-yl)pentanamide; (Chemical Formula: C33H40N4O3), (Molecular Weight: 540.70).

### Metal Ion Sensing:

The procedure for metal ion sensing is described. Solutions of the metal ions (10.0 mM) are prepared in deionized water. A stock solutions of Compounds R1 and R2 (0.2 mM) are each prepared in ethanol and then diluted to 20 µM with ethanol-water (1: 1, v/v, pH 7.04) for spectral measurement. For titration experiments, a 2.0 mL solution of Compounds R1 and R2 (20 µM) are filled in a respective quartz optical cell of 1 cm optical path length. Sn²⁺ stock solution is added into the quartz optical cell gradually by micro-pipette. Spectral data is recorded at 5 min after addition. In selectivity experiments, the test samples are prepared by placing appropriate amounts of metal ion stock into 2.0 mL solution of R1, R2 (20 µM). For fluorescence measurements, excitation is provided at 560 nm, while emission is collected from 565 to 700 nm.

pH titration of Compounds R1 and R2 is described. Stock solutions of Compounds R1 and R2 are respectively added to sodium phosphate buffers of various pH to a final concentration of 10 µM. The fluorescence emission spectra are recorded as a function of pH using λₑₓ at 560 nm. The titration curves are plotted by fluorescence emission intensities at 580 nm versus pH.

Cell culture preparation is described. The KB cell line was provided by of Biochemistry and Cell Biology (China). Cells are grown in MEM (Modified Eagle's Medium) supplemented with 10% FBS (Fetal Bovine serum) and 5% CO₂ at 37° C. Cell (5 × 10⁻⁸ L⁻¹) are plated on 18 nm glass coverslips and allowed to adhere for 24 hours. The *Streptococcus mutans* (ATCC® 700610™) is prepared by inoculating the single colony from the BHI (Brain-Heart Infusion) agar plate into 5 mL BHI broth and incubating at 37°C for 48 h.

Fluorescence imaging is described. Confocal fluorescence imaging is performed with an OLYMPUS IX81 laser scanning microscope and a 60 × oil immersion objective lens. The microscope is equipped with multiple visible laser lines (405, 488, 543 nm). Images are collected and processed with Olympus FV10-ASW software. For fluorescence imaging of intracellular Sn²⁺: 10 µM of Compound R1 or R2 in the culture media containing 0.2% (v/v) DMSO (Dimethyl sulfoxide) is added to the cells. The cells are incubated at 37 °C for 30 min, and washed with PBS three times to remove the excess probe and bathed in PBS (2 mL) before imaging. After washing with PBS (2 mL × 3) to remove the excess probe, the cells are treated with 50 µM SnF₂ for 30 min. Excitation of R1 or R2 loaded cells at 543 nm is carried out with a semiconductor laser, and emission is collected at 560-660 nm (single channel). Alternatively, 50 µM SnF₂ in the culture media is added to the cells. The cells are incubated at 37 °C for 30 min, and washed with PBS three times to remove the excess SnF₂. After washing with PBS (2 mL × 3) to remove the excess SnF₂, the cells are treated with 10 µM R1 or R2 separately for 30 min, and washed with PBS three times to remove the excess probe and bathed in PBS (2 mL) before imaging. Cell imaging is then carried out as the former.

Colocalization experiments are described. 50 µM SnF₂ in the culture media is added to the cells. The cells are incubated at 37 °C for 30 min, and washed with PBS three times to remove the excess SnF₂. After washing with PBS (2 mL × 3) to remove the excess SnF₂, the cells are treated with 10 µM of Compound R1 or R2 separately for 30 min, and washed with PBS three times to remove the excess probe and bathed in PBS (2 mL) before imaging. Cell imaging is then carried out as the former. After washing with PBS (2 mL × 3) to remove the excess probes, the cells were treated with 1.0 µM LysoTracker® Green DND at 37 °C for 30 min. Cell imaging is then carried out as the former.

For fluorescence imaging of Sn²⁺ in bacteria is described. Freshly diluted *Streptococcus mutans* (ATCC® 700610™) is sub cultured in the presence of the 10 µM Compound R1 or R2, separately at 37°C on a shaker bed at 400 rpm for 60 min. Then the bacteria are collected by centrifugation at 8,000 rpm for 2 min and rinsed with Saline (pH = 7.0). The process is repeated three times before imaging. After washing with Saline (2 mL × 3) to remove the excess probes, the bacteria is cultured in the presence of the 50 µM SnF₂ at 37 °C on a shaker bed at 400 rpm for 60 min. Then the bacteria are collected by centrifugation at 8,000 rpm for 2 min and rinsed with Saline (pH = 7.0). The process was repeated three times before imaging. The light source is at = 543 nm provided excitation and emission are collected in the range = 560-660 nm.

Metal ion response is described. Fluorescent 'turn on' probe is conducive for detection target. The solution of Compound R1 or R2 (20 µM) in ethanol-water (1: 1, v/v, PH 7.04) is non-fluorescent. With addition of Sn²⁺ (0-20 eq), fluorescence at 580 nm is turned on and grown drastically with an excitation of 560 nm (Fig 1a and 1c) due to the ring open reaction of rhodamine induced by Sn²⁺ chelating. High-level selectivity is of paramount importance for an excellent chemosensor. Compounds R1 and R2 show selectivity on sensing Sn²⁺. The solution of R1 and R2 (20 µM) in ethanol-water (1: 1, v/v, PH 7.04) are tuning on just in the presence of Sn²⁺ and Cr³⁺, while other transition and heavy metal ions such as K⁺, Ag⁺, Ca²⁺, Mg²⁺, Zn²⁺, Pb²⁺, Ni²⁺, Mn²⁺, Co²⁺, Cd²⁺, Hg²⁺, displayed minimal enhancement with an excitation of 560 nm (Fig 1b and 1d). Metal ion response of R1 and R2 are suited for detection of Sn²⁺ in living *Streptococcus mutans* cells.

Figure 1 (a) and (c) show the fluorescence spectra of comparative Compound R1 and inventive Compound R2 (20 µM) in ethanol-water (1: 1, v/v, pH 7.04) upon addition of 0-20 eq of Sn²⁺. Figure 1 (b) and (d) show the fluorescent spectra of Compounds R1 and R2 (2.0 × 10⁻⁵ M) upon various metal ions (20.0 × 10⁻⁵ M) in ethanol-water (1: 1, v/v, pH 7.04) with an excitation of 560 nm.

The impact of pH values on fluorescence is described. The pirolactam ring of the rhodamine derivatives will open in a certain pH range and indicates the fluorescence of rhodamine. It is therefore necessary to check the fluorescence properties of Compounds R1 and R2 in solutions with different pH values. Furthermore, in the cell, the acidity of different organelles may vary greatly. For example, the normal pH of lysosomes is 4.5-5.5, which may induce ring opening of R1 or R2. Considering the application of Sn²⁺ probe R1 and R2 intracellular or extracellular may be disturbed by the pH, the acid-base titration experiments are carried out by adjusting the pH with an aqueous solution of NaOH and HCl in Phosphate-Buffered Saline ("PBS") (Fig.2 a and b). The titration revealed that the pH range for inducing Compounds R1 or R2 fluorescence turning on is 2.5-6 or 2-4.5, respectively. It is predicted that R1 will be turned on by lysosomes in cell without Sn²⁺ present. Figures 2a and 2b show the dependence of fluorescence at 580 nm of comparative Compound R1 and inventive Compound R2 (10 µM) at different pH in PBS solution. Excitation was at 560 nm.

Fluorescence imaging of intracellular Sn²⁺ is described. Sn²⁺ is usually added to toothpaste, so oral epithelial cells are most likely to come into contact with Sn²⁺. The KB cells are a good candidate for explored Sn²⁺ distribution in cell level by fluorescence imaging. Here the practical applicability of R1 and R2 as a Sn²⁺ probe in the fluorescence imaging of living KB cells is investigated. Firstly, the KB cells are separately stained with 10 µM of Compound R1 or R2 at 37 °C for 30 min. As determined by laser scanning confocal microscopy, R1 gave fluorescence emission in a site of KB cells without Sn²⁺ present (fig 3 .b1); R2 gave scarcely fluorescence (fig 3. b2). This result is consistent with the pH titration experiment that lysosomes acidity may induce R1 fluorescence emission. R2 gave scarcely fluorescence due its lower pH response. This demonstrates the superiority of R2 over R1 given the greater diversity of pH environments that R2 may be used, and subsequently less background noise, particularly in more acidic environments like lysosomes.

Furthermore, when the cells are supplemented with Compound R1 or R2 in the PBS for 30 min at 37 °C and then incubated with 50 µM Sn²⁺ under the same conditions, inventive Compound R2 gave a significant fluorescence increasing from the certain intracellular region (Fig 3 .c2, f2) whereas comparative Compound R1 showed slightly changing in fluorescence intensity (fig 3 .c1, f1). Accordingly, cell imaging experiment indicate that R2 is more suited for detection Sn²⁺ at a cell level. Furthermore, R1 and R2 may be specificity targeting for lysosomes, due to R1 and R2 bear the groups similar to 'dimethylethylamino' that is the targeting anchor for lysosomes.

Figures 3a and 3b show simplified illustrations derived based upon CLSM images of KB cells (a1-c1) and (a2-c2). Cells separately incubated with 10µM of Compounds R1 and R2 for 30 min, (d1-f1) and (d2-f2) followed incubated with 50µM SnF₂ for 30 min. Emission was collected in red channel at 560-660 nm (b1, e1, b2, e2); a1, d1, a2, d2 are bright field images and c1, f1, c2, f2 are overlay images, respectively (λex = 543 nm). Any observed fluorescence is red.

Figure 4 shows simplified illustrates derived based upon CLSM images of KB cells of a comparative colocalization experiment. Firstly, the KB cells are stained with 50 µM Sn²⁺ at 37° C for 30 min, and then separately incubated with 10 µM of Compound R1 and 1µM LysoTracker® Green DND or Compound R2 and 1 µM LysoTracker® Green DND under the same conditions. As determined by laser scanning confocal microscopy, R1 and R2 give fluorescence emission in a site of KB cells (Fig. 4 by, b2), which overlap with LysoTracker® Green DND very well (Fig. 4 d1, b2). There is no fluorescence in other intracellular sites. This surprising result indicates that Sn²⁺ is internalized into cells and leads to accumulation of the ions in lysosomes. This may be a transmission path of Sn. Accordingly, inventive Compound R2 is superior over R1 in this application. Many pharmaceutical agents, including various large and small molecules, must be delivered specifically to particular cell organelles in order to efficiently exert their therapeutic action. Such delivery is still mainly an unresolved problem, but targeting detection is helpful attempt.

Figure 4 shows simplified illustrations derived based upon CLSM images of KB cells of a comparative colocalization experiment (a1 - d1). Cells are successively incubated with 50µM SnF₂, 10 µM of comparative Compound R1 and 1µM LysoTracker® Green DND each for 30 min; (a2-d2) successively incubated with 50µM SnF₂, 10µM of inventive Compound R2 and 1µM LysoTracker® Green DND, each for 30 min. Emission is collected in red channel (b1, b2) at 560-660 nm (λex = 543 nm) or in green channel at 500-540 nm (λex = 488 nm); a1, a2, are bright field images and d1, d2 are overlay images, respectively.

Turning to Figures 5a and 5b, fluorescence imaging of Sn²⁺ in bacteria is described. Figures 5a and 5b show simplified illustrations derived based upon CLSM. The illustrations are not drawn to scale but rather enlarged for illustrative purposes. It has been reported that Sn²⁺ can inhibit metabolism of *Streptococcus mutans.* The practical applicability of Compounds R1 and R2 as a Sn²⁺ probe in the fluorescence imaging of living *Streptococcus mutans* (ATCC® 700610™) are investigated. Firstly, the *Streptococcus mutans* (ATCC® 700610™) are separately stained with 10µM Compound R1 or R2 at 37° C for 60 min. As determined by laser scanning confocal microscopy, Compounds R1 and R2 gave no fluorescence emission without Sn²⁺ present (Fig. 5a and 5b; b1 and b2, respectively). When the *Streptococcus mutans* are supplemented with R1 or R2 in the PBS for 60 min at 37° C and then incubated with 50 µM Sn²⁺ under the same conditions, R1 and R2 gave a significant fluorescence increasing (Fig. 5a and 5b; e1 and e2, respectively). The overlay of fluorescence and Brightfield images revealed that the fluorescence signals are localized in the *Streptococcus mutans* (ATCC® 700610™) (Figure 5a and 5b; f1 andf2, respectively), indicating that the Sn²⁺ plays its physiological role within bacteria. This data provides evidence for antibacterial mechanism of Sn²⁺.

Figures 5a and 5b show simplified illustrations derived based upon CLSM images of *Streptococcus mutans* (ATCC® 700610™). The illustrations are not drawn to scale but rather enlarged for illustrative purposes. (a1-c1) and (a2-c2) Cells are separately incubated with 10µM comparative Compound R1 and inventive Compound R2 for 30 min, (d1-f1) and (d2-f2) followed incubated with 50µM SnF₂ for 30 min. Emission is collected in red channel at 560-660 nm (b1, e1, b2, e2); a1, d1, a2, d2 are bright field images and c1, f1, c2, t2 are overlay images, respectively (λex = 543 nm).

In summary, the biological application of inventive Compound R2 is demonstrated by the imaging of Sn²⁺ in KB cells and *Streptococcus mutans* (ATCC® 700610™). Furthermore compound R2 as lysosomes tracker is demonstrated by the distribution of Sn²⁺ in cells and bacteria, which is helpful to research of pharmaceutical agents' delivery and antibacterial mechanism of Sn²⁺.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A compound of the following Formula (I): 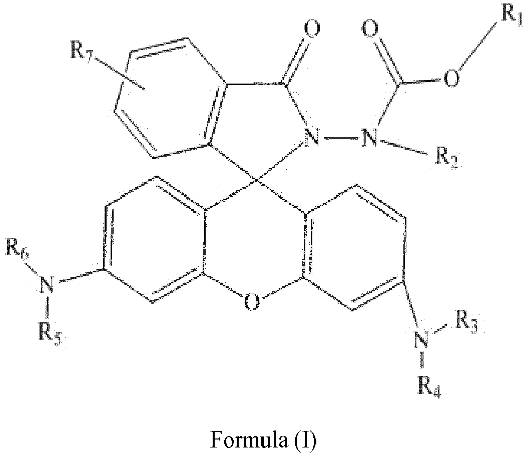 wherein R₁ is unsubstituted, branched or unbranched C₁-C₁₂ alkyl, alkenyl, or alkynyl; and wherein R₂, R₃, R₄, R₅, R₆, and R₇ are each independently a hydrogen or a hydrocarbyl, heteroalkyl, heteroalkenyl, heteroalkynyl, heterocycloalkyl, heterocycloalkenyl, heteroaryl, and wherein the aforementioned may be substituted or unsubstituted;
or an optical isomer, diastereomer or enantiomer for Formula (I), or a salt thereof.

2. The compound of claim 1, wherein R₂, R₃, R₄, R₅, R₆, and R₇ are each independently selected from the group consisting of H, alkyl, alkenyl, alkynyl, cycloalkyl, cycloalkenyl, aryl.

3. The compound of claim 1 or 2, wherein: R₂, R₃, R₄, R₅, R₆, and R₇ are each independently selected from H, C₁-C₁₀ alkyl or alkenyl, and wherein the aforementioned may be substituted or unsubstituted.

4. The compound of any one of the preceding claims, wherein:
R₂ is hydrogen;
R₃, R₄, R₅, and R₆ are each independently selected from hydrogen, or unsubstituted C₁-C₅ alkyl, branched or unbranched; and
R₇ is hydrogen.

5. The compound of any one of the preceding claims, wherein R₁ is unsubstituted, branched or unbranched C₁-C₁₀ alkyl.

6. A compound of the following Formula (II): 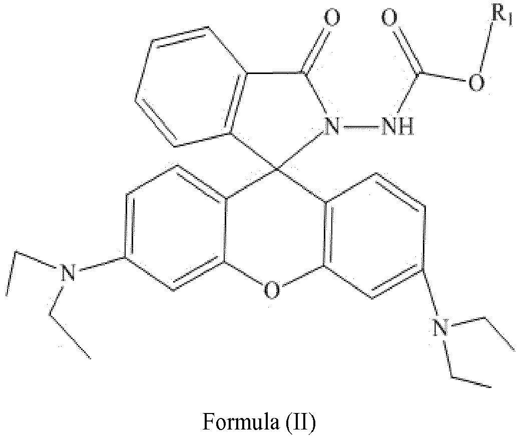 wherein R₁ is unsubstituted, branched or unbranched C₁-C₁₂ alkyl or alkenyl;
or an optical isomer, diastereomer or enantiomer for Formula (I), or a salt thereof.

7. The compound of claim 6, wherein R₁ is a C₁-C₁₀ alkyl, thereof.

8. The compound of claim 6 or 7, wherein R₁ is C₁-C₈ alkyl.

9. The compound of claim 1 or 6, wherein the compound is selected from the group consisting of:
(a) tert-butyl (3',6'-diamino-3-oxospiro[isoindoline-1,9'-xanthen]-2-yl)carbamate;
(b) tert-butyl (3',6'-bis(dimethylamino)-3-oxospiro[isoindoline-1,9'-xanthen]-2-yl)carbamate;
(c) tert-butyl (3',6'-bis(diethylamino)-3-oxospiro[isoindoline-1,9'-xanthen]-2-yl)carbamate;
(d) tert-butyl (3',6'-bis(ethylamino)-2',7'-dimethyl-3-oxospiro [isoindoline-1,9'-xanthen]-2-yl)carbamate;
(e) tert-butyl (3',6'-diamino-2',7'-dimethyl-3-oxospiro[isoindoline-1,9'-xanthen]-2-yl)carbamate;
(f) tert-butyl (3-oxo-3',6'-di(pyrrolidin-1-yl)spiro[isoindoline-1,9'-xanthen]-2-yl)carbamate;
(g) tert-butyl (3-oxo-3',6'-bis(phenylamino)spiro[isoindoline-1,9'-xanthen]-2-yl)carbamate;
(h) tert-butyl (3-oxo-3',6'-di(piperidin-1-yl)spiro[isoindoline-1,9'-xanthen]-2-yl)carbamate;
(i) tert-butyl (3',6'-dimorpholino-3-oxospiro[isoindoline-1,9'-xanthen]-2-yl)carbamate;
(j) tert-butyl(2',7'-dibutyl-3',6'-bis(diethylamino) -3-oxospiro[isoindoline-1,9'-xanthen]-2-yl)carbamate;
(k) tert-butyl (2',7'-dimethyl-3-oxo-3',6'-di(piperidin-1-yl)spiro [isoindoline-1,9'-xanthen]-2-yl)carbamate;
(l) tert-butyl (3-oxo-1',2',3',4',10',11',12',13'-octahydrospiro[isoindoline-1,7'-pyrano [2,3-f:6,5-f']diquinolin]-2-yl)carbamate;
(m) tert-butyl (3-oxo-1',2',3',4',8',9',10',11'-octahydrospiro[isomdolme-1,6'-pyrano [3,2-g:5,6-g']diquinolin]-2-yl)carbamate;
(n) N-(3',6'-bis(diethylamino)-3-oxospiro[isoindoline-1,9'-xanthen]-2-yl)propionamide;
(p) N-(3',6'-bis(diethylamino)-3-oxospiro[isoindoline-1,9'-xanthen]-2-yl)butyramide; and
(q) N-(3',6'-bis(diethylamino)-3-oxospiro[isoindoline-1,9'-xanthen]-2-yl)pentanamide.

10. The compound of claim 1, wherein the compound is selected from the group consisting of:
(a) Tert-butoxy-carboxamide, N-[3',6'-bis(diethylamino)-3-oxospiro [1H-isoindole-1,9'-[9H]xanthen]-H)-yl]-;
(b)Tert-butoxy-carboxamide,N-[3',6'-bis(dimethylamino)-3-oxospiro[1H-isoindole-1,9'-[9H]xanthen]-2(3H)-yl]-;
(c)Methoxy-carboxamide,N-[3',6'-bis(diethylamino)-3-oxospiro[1H-isoindole-1,9'-[9H]xanthen]-2(3H)-yl]-;
(d)Ethoxy-carboxamide,N-[3',6'-bis(diethylamino)-3-oxospiro[1H-isoindole-1,9'-[9H]xanthen]-2(3H)-yl]-;
(e)Methoxy-carboxamide,N-[3',6'-bis(dimethylamino)-3-oxospiro[1H-isoindole-1,9'-[9H]xanthen]-2(3H)-yl]-; and
(f)Ethoxy-carboxamide,N-[3',6'-bis(dimethylamino)-3-oxospiro[1H-isoindole-1,9'-[9H]xanthen]-2(3H)-yl]-.

11. A method of detecting fluorescence in a biological cell comprising the steps:
(a) incubating the cell with a compound of any one of the preceding claims;
(b) shining excitation light to the incubated cell;
(c) detecting light emission from the compound from 560 nm to 660 nm.

12. The method of claim 11, subjecting the biological cell to Sn²⁺.

13. The method of claim 11 or 12, wherein the biological cell is selected from an oral epithelial cell or a *Streptococcus* genus of bacterium

14. The method of claim 11 or 12, wherein the biological cell is a eukaryotic cell, and wherein preferably said emission detection is a lysosome.

15. The method of any one of claims 12-14, wherein the compound is a compound of claim 9 or 10, preferably wherein the compound is of claim 10.

## Patentansprüche

1. Verbindung der folgenden Formel (I): 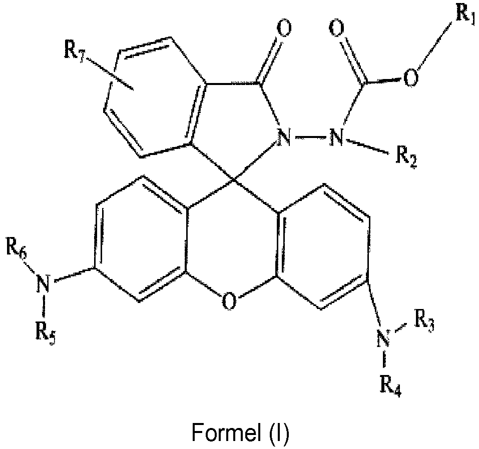 wobei R₁ für unsubstituiertes, verzweigtes oder unverzweigtes C₁-C₁₂-Alkyl, -Alkenyl oder-Alkinyl steht; und wobei R₂, R₃, R₄, R₅, R₆, und R₇ jeweils unabhängig für einen Wasserstoff oder ein Hydrocarbyl, Heteroalkyl, Heteroalkenyl, Heteroalkinyl, Heterocycloalkyl, Heterocycloalkenyl, Heteroaryl stehen und wobei die Vorgenannten substituiert oder unsubstituiert sein können;
oder ein optisches Isomer, Diastereomer oder Enantiomer der Formel (I), oder ein Salz davon.

2. Verbindung nach Anspruch 1, wobei R₂, R₃, R₄, R₅, R₆, und R₇ jeweils unabhängig ausgewählt sind aus der Gruppe bestehend aus H, Alkyl, Alkenyl, Alkinyl, Cycloalkyl, Cycloalkenyl, Aryl.

3. Verbindung nach Anspruch 1 oder 2, wobei: R₂, R₃, R₄, R₅, R₆, und R₇ jeweils unabhängig ausgewählt sind aus H, C₁-C₁₀-Alkyl oder -Alkenyl, und wobei die Vorgenannten substituiert oder unsubstituiert sein können.

4. Verbindung nach einem der vorgenannten Ansprüche, wobei:
R₂ Wasserstoff ist;
R₃, R₄, R₅, und R₆ jeweils unabhängig ausgewählt sind aus Wasserstoff oder unsubstituiertem C₁-C₅-Alkyl, verzweigt oder unverzweigt; und
R₇ für Wasserstoff steht.

5. Verbindung nach einem der vorgenannten Ansprüche, wobei R₁ für unsubstituiertes, verzweigtes oder unverzweigtes C₁-C₁₀-Alkyl steht.

6. Verbindung der folgenden Formel (II): 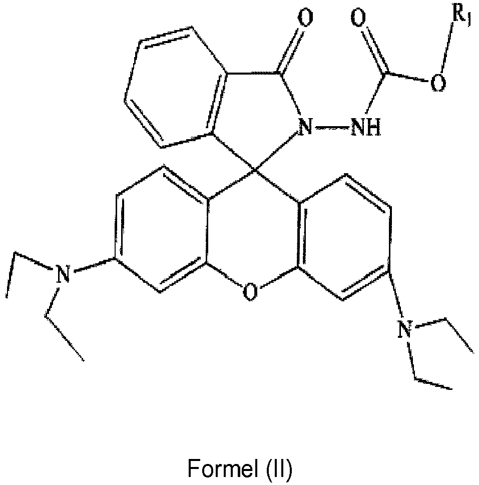 wobei R₁ für unsubstituiertes, verzweigtes oder unverzweigtes C₁-C₁₂-Alkyl oder -Alkenyl steht;
oder ein optisches Isomer, Diastereomer oder Enantiomer der Formel (I), oder ein Salz davon.

7. Verbindung nach Anspruch 6, wobei R₁ davon für ein C₁-C₁₀-Alkyl steht.

8. Verbindung nach Anspruch 6 oder 7, wobei R₁ für C₁-C₈-Alkyl steht.

9. Verbindung nach Anspruch 1 oder 6, wobei die Verbindung aus der Gruppe ausgewählt ist bestehend aus:
(a) tert.-Butyl-(3',6'-diamino-3-oxospiro[isoindolin-1,9'-xanthen]-2-yl)carbamat;
(b) tert.-Butyl-(3',6'-bis(dimethylamino)-3-oxospiro[isoindolin-1,9'-xanthen]-2-yl)-carbamat;
(c) tert.-Butyl-(3',6'-Bis(diethylamino)-3-oxospiro[isoindolin-1,9'-xanthen]-2-yl)-carbamat;
(d) tert.-Butyl-(3',6'-bis(ethylamino)-2',7'-dimethyl-3-oxospiro[isoindolin-1,9'-xanthen]-2-yl)carbamat;
(e) tert.-Butyl-(3',6'-diamino-2',7'-dimethyl-3-oxospiro[isoindolin-1,9'-xan-then]-2-yl)carbamat;
(f) tert.-Butyl-(3-oxo-3',6'-di(pyrrolidin-1-yl)spiro[isoindolin-1,9'-xanthen]-2-yl)carbamat;
(g) tert.-Butyl-(3-oxo-3',6'-bis(phenylamino)spiro[isoindolin-1,9'-xanthen]-2-yl)-carbamat;
(h) tert.-Butyl-(3-oxo-3',6'-di(piperidin-1-yl)spiro[isoindolin-1,9'-xanthen]-2-yl)carbamat;
(i) tert.-Butyl-(3',6'-dimorpholino-3-oxospiro[isoindolin-1,9'-xanthen]-2-yl)carbamat;
(j) tert.-Butyl-(2',7'-dibutyl-3',6'-Bis(diethylamino)-3-oxospiro[isoindolin-1,9'-xanthen]-2-yl)carbamat;
(k) tert.-Butyl-(2',7'-dimethyl-3-oxo-3',6'-di(piperidin-1-yl)spiro[isoindolin-1,9'-xanthen]-2-yl)carbamat;
(l) tert.-Butyl-(3-oxo-1',2',3',4',10',11',12',13'-octahydrospiro[isoindolin-1,7'-pyrano[2,3-f:6,5-f']dichinolin]-2-yl)carbamat;
(m) tert.-Butyl(3-oxo-1',2',3',4',8',9',10',11'-octahydrospiro[isoindolin-1,6'-pyrano[3,2-g:5,6-g']dichinolin]-2-yl)carbamat;
(n) N-(3',6'-Bis(diethylamino)-3-oxospiro[isoindolin-1,9'-xanthen]-2-yl)propion-amid;
(p) N-(3',6'-Bis(diethylamino)-3-oxospiro[isoindolin-1,9'-xanthen]-2-yl)butyr-amid; und
(q) N-(3',6'-Bis(diethylamino)-3-oxospiro[isoindolin-1,9'-xanthen]-2-yl)pentan-amid.

10. Verbindung nach Anspruch 1, wobei die Verbindung ausgewählt ist aus der Gruppe bestehend aus:
(a) tert.-Butoxy-carboxamid, N-[3',6'-bis(diethylamino)-3-oxospiro[1H-isoindol-1,9'-[9H]xanthen]-H)-yl]-;
(b) tert.-Butoxy-carboxamid,N-[3',6'-bis(dimethylamino)-3-oxospiro[1 H-isoindol-1,9'-[9H]xanthen]-2(3H)-yl]-;
(c) Methoxy-carboxamid,N-[3',6'-bis(diethylamino)-3-oxospiro[1H-isoindol-1,9'-[9H]xanthen]-2(3H)-yl]-;
(d) Ethoxy-carboxamid,N-[3',6'-bis(diethylamino)-3-oxospiro[1H-isoindol-1,9'-[9H]xanthen]-2(3H)-yl]-;
(e) Methoxy-carboxamid, N-[3',6'-bis(dimethylamno)-3-oxospiro[1H-isoindol-1,9'-[9H]xanthen]-2(3H)-yl]-; und
(f) Ethoxy-carboxamid,N-[3',6'-bis(dimethylamino)-3-oxospiro[1H-isoindol-1,9'-[9H]xanthen]-2(3H)-yl]-.

11. Verfahren zum Nachweis von Fluoreszenz in einer biologischen Zelle, umfassend die Schritte:
(a) Inkubieren der Zelle mit einer Verbindung nach einem der vorgenannten Ansprüche;
(b) Bestrahlen der inkubierten Zelle mit Anregungslicht;
(c) Nachweisen der Lichtemission von 560 nm bis 660 nm aus der Verbindung.

12. Verfahren nach Anspruch 11, Aussetzen der biologischen Zelle gegenüber Sn²⁺.

13. Verfahren nach Anspruch 11 oder 12, wobei die biologische Zelle ausgewählt ist aus einer Mundepithelzelle oder einem Bacterium der Gattung *Streptococcus.*

14. Verfahren nach Anspruch 11 oder 12, wobei die biologische Zelle eine eukaryotische Zelle ist, und wobei der Emissionsnachweis vorzugsweise ein Lysosom ist.

15. Verfahren nach einem der Ansprüche 12-14, wobei die Verbindung eine Verbindung nach Anspruch 9 oder 10 ist, vorzugsweise wobei die Verbindung nach Anspruch 10 ist.

## Revendications

1. Composé de formule (I) suivante : 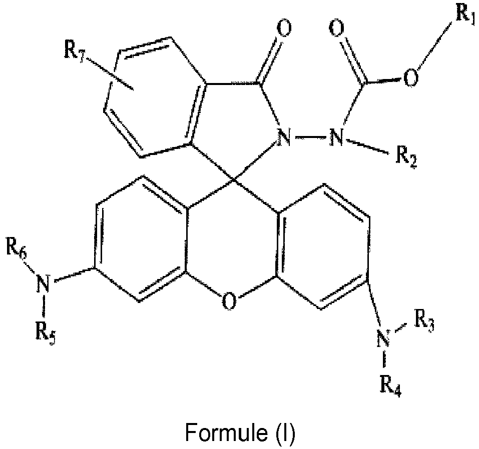 dans lequel R₁ est un alkyle, alcényle ou alcynyle, non substitué, ramifié ou non ramifié, en C₁ à C₁₂ ; et dans lequel R₂, R₃, R₄, R₅, R₆ et R₇ sont chacun indépendamment un hydrogène ou un hydrocarbyle, un hétéroalkyle, un hétéroalcényle, un hétéroalcynyle, un hétérocycloalkyle, un hétérocycloalcényle, un hétéroaryle, et dans lequel les susmentionnés peuvent être substitués ou non substitués ;
ou un isomère optique, diastéréo-isomère ou énantiomère pour la Formule (I), ou un sel de celui-ci.

2. Composé selon la revendication 1, dans lequel R₂, R₃, R₄, R₅, R₆ et R₇ sont chacun indépendamment choisis parmi le groupe constitué de H, alkyle, alcényle, alcynyle, cycloalkyle, cycloalcényle, aryle.

3. Composé selon la revendication 1 ou 2, dans lequel : R₂, R₃, R₄, R₅, R₆ et R₇ sont chacun indépendamment choisis parmi H, un alkyle ou alcényle en C₁ à C₁₀, et dans lequel le radical susmentionné peut être substitué ou non substitué.

4. Composé selon l'une quelconque des revendications précédentes, dans lequel :
R₂ est un hydrogène ;
R₃, R₄, R₅ et R₆ sont chacun indépendamment choisis parmi un hydrogène, ou un alkyle non substitué en C₁ à C₅, ramifié ou non ramifié ; et
R₇ est un hydrogène.

5. Composé selon l'une quelconque des revendications précédentes, dans lequel R₁ est un alkyle non substitué, ramifié ou non ramifié en C₁ à C₁₀.

6. Composé de formule (II) suivante : 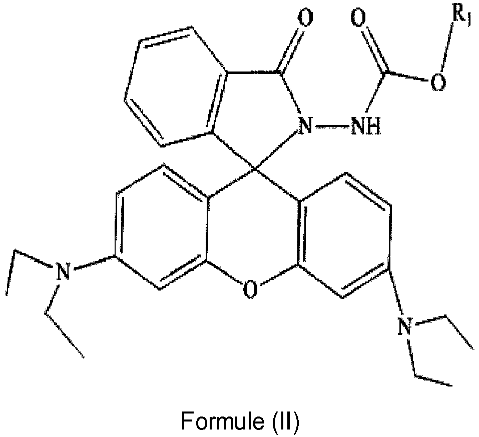 dans lequel R₁ est un alkyle ou alcényle non substitué, ramifié ou non ramifié en C₁ à C₁₂ ;
ou un isomère optique, diastéréo-isomère ou énantiomère pour la Formule (I), ou un sel de celui-ci.

7. Composé selon la revendication 6, dans lequel R₁ est un alkyle en C₁-C₁₀, de celui-ci.

8. Composé selon la revendication 6 ou 7, dans lequel R₁ est un alkyle en C₁ à C₈.

9. Composé selon la revendication 1 ou 6, où le composé est choisi dans le groupe constitué de :
(a) (3',6'-diamino-3-oxospiro[iso-indoline-1,9'-xanthén]-2-yl)carbamate de tert-butyle ;
(b) (3',6'-bis(diméthylamino)-3-oxospiro[iso-indoline-1,9'-xanthén]-2-yl)carbamate de tert-butyle ;
(c) (3',6'-bis(diéthylamino)-3-oxospiro[iso-indoline-1,9'-xanthén]-2-yl)carbamate de tert-butyle ;
(d) (3',6'-bis(éthylamino)-2',7'-diméthyl-3-oxospiro[iso-indoline-1,9'-xanthén]-2-yl)carbamate de tert-butyle ;
(e) (3',6'-diamino-2',7'-diméthyl-3-oxospiro[iso-indoline-1,9'-xanthén]-2-yl)carbamate de tert-butyle ;
(f) (3-oxo-3',6'-di(pyrrolidin-1-yl)spiro[iso-indoline-1,9'-xanthén]-2-yl)carbamate de tert-butyle ;
(g) (3-oxo-3',6'-bis(phénylamino)spiro[iso-indoline-1,9'-xanthén]-2-yl)carbamate de tert-butyle ;
(h) (3-oxo-3',6'-di(pipéridin-1-yl)spiro[iso-indoline-1,9'-xanthén]-2-yl)carbamate de tert-butyle ;
(i) (3',6'-dimorpholino-3-oxospiro[iso-indoline-1,9'-xanthén]-2-yl)carbamate de tert-butyle ;
(j) (2',7'-dibutyl-3',6'-bis(diéthylamino)-3-oxospiro[iso-indoline-1,9'-xanthén]-2-yl)carbamate de tert-butyle ;
(k) (2',7'-diméthyl-3-oxo-3',6'-di(pipéridin-1-yl)spiro[iso-indoline-1,9'-xanthén]-2-yl)carbamate de tert-butyle ;
(l) (3-oxo-1',2',3',4',10',11',12',13'-octahydrospiro[iso-indoline-1,7'-pyrano[2,3-f:6,5-f']diquinolin]-2-yl)carbamate de tert-butyle ;
(m) (3-oxo-1',2',3',4',8',9',10',11'-octahydrospiro[iso-indoline-1,6'-pyrano[3,2-g:5,6-g']diquinolin]-2-yl)carbamate de tert-butyle ;
(n) N-(3',6'-bis(diéthylamino)-3-oxospiro[iso-indoline-1,9'-xanthén]-2-yl)propionamide ;
(p) N-(3',6'-bis(diéthylamino)-3-oxospiro[iso-indoline-1,9'-xanthén]-2-yl)butyramide ; et
(q) N-(3',6'-bis(diéthylamino)-3-oxospiro[iso-indoline-1,9'-xanthén]-2-yl)pentanamide.

10. Composé selon la revendication 1, où le composé est choisi dans le groupe constitué de :
(a) Tert-butoxy-carboxamide, N-[3',6'-bis(diéthylamino)-3-oxospiro[1 H-isoindole-1,9'-[9H]xanthén]-H)-yl]-;
(b) Tert-butoxy-carboxamide,N-[3',6'-bis(diméthylamino)-3-oxospiro[1 H-isoindole-1,9'-[9H]xanthén]-2(3H)-yl]- ;
(c) Méthoxy-carboxamide,N-[3',6'-bis(diéthylamino)-3-oxospiro[1H-isoindole-1,9'-[9H]xanthén]-2(3H)-yl]- ;
(d) Éthoxy-carboxamide,N-[3',6'-bis(diéthylamino)-3-oxospiro[1H-isoindole-1,9'-[9H]xanthén]-2(3H)-yl]-;
(e) Méthoxy-carboxamide,N-[3',6'-bis(diméthylamino)-3-oxospiro[1H-isoindole-1,9'-[9H]xanthén]-2(3H)-yl]-; et
(f) Éthoxy-carboxamide,N-[3',6'-bis(diméthylamino)-3-oxospiro[1 H-isoindole-1,9'-[9H]xanthén]-2(3H)-yl]-.

11. Procédé de détection de fluorescence dans une cellule biologique comprenant les étapes suivantes :
(a) incubation de la cellule avec un composé selon l'une quelconque des revendications précédentes ;
(b) envoi d'une lumière d'excitation sur la cellule incubée ;
(c) détection d'une émission de lumière à partir du composé de 560 nm à 660 nm.

12. Procédé selon la revendication 11, en soumettant la cellule biologique à Sn²⁺.

13. Procédé selon la revendication 11 ou 12, dans lequel la cellule biologique est choisie parmi une cellule épithéliale buccale ou un genre de bactérie *Streptococcus.*

14. Procédé selon la revendication 11 ou 12, dans lequel la cellule biologique est une cellule eucaryote, et dans lequel, de préférence, ladite détection d'émission est un lysosome.

15. Procédé selon l'une quelconque des revendications 12 à 14, dans lequel le composé est un composé selon la revendication 9 ou 10, de préférence dans lequel le composé est selon la revendication 10.
